# EUROPEAN PATENT SPECIFICATION

(11) **EP 1 392 323 B1**
(45) Date of publication and mention of the grant of the patent: **08.10.2008**
(21) Application number: 02740584.4
(22) Date of filing: 10.05.2002
(51) Int. Cl.: A61K 31/593, A61P 37/02

(54) **USE OF A VITAMIN D3 ANALOGUE FOR THE TREATMENT OF AUTOIMMUNE DIABETES**
VERWENDUNG EINES ANALOGES DES VITAMINS D3 ZUR BEHANDLUNG DES AUTOIMMUNEN DIABETES
UTILISATION D'un ANALOGUE DE LA VITAMINE D3 POUR LE TRAITEMENT Du DIABETE AUTO-IMMUNe

(30) Priority: 22.05.2001 EP 01112421
(43) Date of publication of application: 03.03.2004
(73) Proprietor: Bioxell S.p.a., 20132 Milano (IT)
(72) Inventor: ADORINI, Luciano, I-20133 Milano (IT); GIARRATANA, Nadia, I-21040 Oggiona S. Stefano (IT); GREGORI, Silvia, I-20090 Buccinasco (IT); SMIROLDO, Simona, I-20122 Milano (IT); USKOKOVIC, Milan, Radoje, Upper Montclair, NJ 07043 (US)
(74) Representative: Wilkinson, Stephen John
(86) International application number: PCT/EP2002/005162
(87) International publication number: WO 2002/094247

(56) References cited:
- EP-A- 0 654 467
- WO-A-98/18468
- WO-A-99/12894
- US-A- 5 665 387
- GREGORI SILVIA ET AL: "A 1alpha,25-dihydroxyvitamin D3 analog enhances regulatory T-cells and arrests autoimmune diabetes in NOD mice." DIABETES, vol. 51, no. 5, May 2002 (2002-05), pages 1367-1374, XP009002961 May, 2002 ISSN: 0012-1797
- MATHIEU CHANTAL ET AL: "The coming of age of 1,25-dihydroxyvitamin D3 analogs as immunomodulatory agents." TRENDS IN MOLECULAR MEDICINE, vol. 8, no. 4, April 2002 (2002-04), pages 174-179, XP002226059 April, 2002 ISSN: 1471-4914
- USKOKOVIC MILAN R ET AL: "Highly active analogs of 1alpha,25-dihydroxyvitamin D3 that resist metabolism through C-24 oxidation and C-3 epimerization pathways." STEROIDS, vol. 66, no. 3-5, March 2001 (2001-03), pages 463-471, XP002226060 ISSN: 0039-128X
- VERSTUYF A ET AL: "Recent developments in the use of vitamin D analogues." EXPERT OPINION ON INVESTIGATIONAL DRUGS. ENGLAND MAR 2000, vol. 9, no. 3, March 2000 (2000-03), pages 443-455, XP009003262 ISSN: 1354-3784

## Description

The present invention is concerned with the use of 1,25-dihydroxy-16,23Z-diene-26,27-hexafluoro-19-nor vitamin D₃ (Compound A) for the manufacture of a medicament for the treatment of autoimmune diabetes, especially type 1 diabetes and for the manufacture of a medicament for the prevention and/or treatment of acute and chronic allograft rejection.

1,25-dihydroxy-16,23Z-diene-26,27-hexafluoro-19-nor vitamin D3 can be administered in an amount effective to prevent and/or to treat such diseases, alone or in combination with other immunosuppressive agents.

1,25-dihydroxyvitamin D₃ [1,25(OH)₂D₃], the activated form of vitamin D₃, is a secosteroid hormone that not only plays a central role in bone and calcium metabolism, but also modulates the immune response via specific receptors expressed in APCs and activated T cells (Bouillon, R., et al., Eur. J. Endocrinol. 1995, 133:7-16). 1,25(OH)₂D₃ and its analogues have been shown to inhibit autoimmune diseases in animal models, such as experimental allergic encephalomyelitis (Lemire, J.M. et al. J. Clin. Invest. 1991, 87:1103-1107; Cantorna, M.T., et al. Proc. Natl. Acad. Sci. U S A. 1996, 93:7861-7864; and Mattner, F., S. et al. Eur. J. Immunol. 2000,30:498-508), murine lupus (Lemire, J.M., et al. Autoimmunity. 1992,12:143-148), collagen-induced arthritis (Larsson, P., L. et al., Clin. Exp. Immunol. 1998,114:277-283; and Cantorna, M.T., et al. J. Nutr. 1998, 128:68-72) and type 1 DM (= *diabetes mellitus*) (Mathieu, C., M. et al. Diabetologia. 1994, 37:552-558). They are clinically used in the treatment of psoriasis, a Th1-mediated autoimmune disease of the skin, where they show an efficacy comparable to topical steroids (Fogh, K., and K. Kragballe, Curr. Pharm. Des. 2000,6:961-972). Polymorphysms of the vitamin D receptor gene have been associated with type 1 DM in different populations (Pani, M.A., et al. Diabetes. 2000, 49:504-507; Chang, T.J., et al. Clin. Endocrinol. (Oxf). 2000, 52:575-580). Epidemiological studies show higher incidence of the disease in northern than in southern latitudes (Green, A., et al. Lancet. 1992, 339:905-909), suggesting a possible involvement of a 1,25(OH)₂D₃ deficiency in the pathogenesis of type 1 DM. This is supported by a large population-based case-control study showing that the intake of vitamin D₃ contributes to a decreased risk in type 1 DM onset (Diabetologia. 1999, 42:51-54).

1,25(OH)₂D₃ inhibits IL-12 production (Lemire, J.M. et al., J. Nutr. 1995, 125:1704S-1708S), likely via inhibition of NF-kB (D'Ambrosio, D., et al. J. Clin. Invest. 1998, 101:252-262), and IL-12 has been shown to exert an important role in the development of type 1 DM in the non-obese diabetic (NOD) mouse (Trembleau, S. et al., J. Exp. Med. 1995, 181:817-821). Recently, the *IL-12B* gene coding for IL-12p40 has been found strongly linked with susceptibility to human type 1 DM (Morahan, G. et al., Nat. Genet. 2001, 27:218-221). Targeting IL-12 may thus prevent disease (Adorini, L., Nat. Genet. 2001, 27:131-132), and 1,25(OH)₂D₃ analogues could represent interesting candidates to inhibit IL-12 production and type 1 DM development.

1,25(OH)₂D₃ and its analogues are immunoregulatory agents able to prevent Th1-mediated autoimmune diseases (Casteels, K., et al. Curr. Opin. Nephrol. Hypertens. 1995, 4:313-318). 1,25(OH)₂D₃ reduces the incidence of insulitis (Mathieu, C., et al., Diabetes. 1992, 41:1491-1495) and prevents type 1 DM development (Mathieu, C., et al. Diabetologia. 1994, 37:552-558), but only when administered to NOD mice starting from three weeks of age, before the onset of insulitis. 1,25(OH)₂D₃ was found ineffective in preventing progression of diabetes in NOD mice when administered from 8 weeks of age, when NOD mice present a well established insulitis (Mathieu, C., et al. Vitamin D and diabetes. Vitamin D, eds. Feldman, Glorieux and Pike. Academic Press. 1997, 1183-1196). However, a combined treatment of 8 week-old NOD mice with the 1,25(OH)₂D₃ analogue MC1288 (see structure below) and cyclosporine A reduced the incidence of disease, although neither treatment alone was effective (Casteels, K.M., et al., A. Endocrinology. 1998,139:95-102).

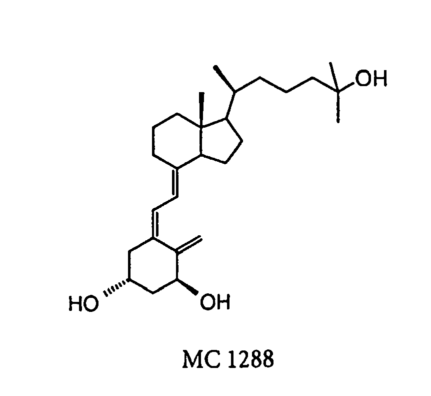

While it is possible to inhibit the onset of autoimmune diabetes by timely administration of 1,25(OH)₂D₃ or analogues thereof it was so far not possible to prevent its progression.

It has now surprisingly been found that the administration of the 1,25(OH)₂D₃ analogue 1,25-dihydroxy-16,23Z-diene-26,27-hexafluoro-19-nor vitamin D₃ to 8 week-old non-obese diabetic (NOD) mice inhibits LPS-induced IL-12 (LPS is lipopolysaccharide, a bacterial product mitogenic for lymphocytes) and IFN-γ production at non-hypercalcemic doses, arrests progression of insulitis and inhibits type 1 DM development. Arrest of disease progression was accompanied by a selective inhibition of IL-2 and IFN-γ production by pancreatic lymph node CD4⁺ T cells. Already a short course of treatment with the 1,25(OH)₂D₃ analogue, 1,25-dihydroxy-16,23Z-diene-26,27-hexafluoro-19-nor vitamin D₃ (Compound A) 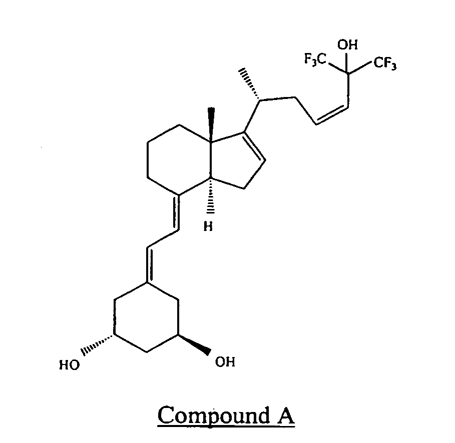 inhibits IL-12 production and enhances the frequency of CD4⁺CD25⁺ regulatory T cells in pancreatic lymph nodes, preventing progression of autoimmune diabetes, especially type 1 diabetes in the NOD mouse.

The invention provides thus the use of 1,25-dihydroxy-16,23Z-diene-26,27-hexafluoro-19-nor vitamin D₃ for the manufacture of medicaments for the prevention of the progression and/or treatment of autoimmune diabetes, especially type 1 diabetes. Autoimmune diabetes, especially type 1 diabetes, can be prevented and/or treated in patients in need of such treatment by administering per os, parenteral (including subcutaneous, intramuscular and intravenous), rectal, buccal (including sublingual), pulmonary, transdermal, and intranasal, most preferably oral, an amount of Compound A effective to prevent the progression and/or treat the disease.

The compound can be used as a monotherapy or it can be administered in combination with known immunosuppressive agents like anti-CD3 antibodies, anti-thymocyte antibodies, cyclosporine A, tacrolimus, rapamicin, CellCept^{®} and Zenapax^{®}, either as inducing agents or in chronic treatment. The combination partner can be admixed with the compound or its salts in various ratios or can be administered separately or sequentially.

In a further embodiment the invention is concerned with the use of Compound A for the manufacture of a medicament for the prevention and/or treatment of acute and chronic allograft rejection. Acute and chronic allograft rejection can be prevented and/or treated by administering Compound A in an amount effective to prevent and/or to treat such rejection, especially, in the case of chronic rejection, for the reducing of the intimal thickening in aortic allografts and decreasing the number of inflammatory cells in the adventitial, media, and intima layer of the grafts. The compound can be used as a monotherapy or it can be administered in combination with known immunosuppressve agents like anti-CD3 antibodies, anti-thymocyte antibodies, cyclosporine A, tacrolimus, rapamicin, CellCept^{®} and Zenapax^{®}, either as inducing agents or in chronic treatment. The combination partner can be admixed with the compound or its salts in various ratios or can be administered separately or sequentially.

1,25-dihydroxy-16,23Z-diene-26,27-hexafluoro-19-nor vitamin D₃ is a known compound and its preparation is described in EP A 0654467.

The present invention is further exemplified by experiments as described below. The following materials and methods are used:

### 1. Mice

The non-obese (NOD)/Lt mice used for the experiments were purchased from Charles River Laboratories (Calco, Italy). All mice were kept under specific pathogen-free conditions. Glucose levels in the tail venous blood were quantified using a EUROFlash (Lifescan, Issy les Moulineaux, France). A diagnosis of diabetes was after two sequential glucose measurements higher than 200 mg/dl.

### 2. Treatments

1,25-dihydroxy-16,23Z-diene-26,27-hexafluoro-19-nor vitamin D₃ was dissolved in ethanol (1 mg/ml) and then diluted in miglyol 812.NOD/Lt. The mice were dosed orally with vehicle (miglyol 812) alone or vehicle containing Compound A (0,03 µg/Kg body weight) 5x/week from 8 to 12 or from 8 to 16 weeks of age.

### 3. Immunohistology

Pancreata were snap-frozen in Tissue Tek (Miles Laboratories, Elkhart, IN), and 5-µm-thick sections stained with hematoxilin and eosin. Insulitis score was quantified as follows: 0, no insulitis; 1, peri-insulitis; 2, insulitis in <50% of the islet; 3, insulitis in >50% of the islet. A mean score was calculated from 40-50 islets per individual pancreas. In addition, pancreas cryostat sections were stained with biotinylated mAb directed against CD4, CD8, B220, CD11b (all purchased from PharMingen), or CD11c (N418; American Type Culture Collection, Manassas, VA), followed by streptavidin-peroxidase conjugate. 3-amino-9-ethylcarbazole (Dako, Carpenteria, CA) was used as chromogen and hematoxylin as a counterstain. For insulin pancreas cryostat sections were 5 µm thick, were stained with anti-porcine insulin (Sigma) followed by PAP (Sigma). 3-amino-9-ethylcarbazole (Dako, Carpenteria, CA) was used as chromogen and hematoxylin as a counterstain.

### 4. Isolation of pancreas-infiltrating T cells

After removal of all visible pancreatic lymph nodes, pancreata were individually digested in 3 ml HBSS containing 1 mg/ml collagenase IV (Sigma), by shaking (200 rpm) at 37°C for 15 min. Single cell suspensions were collected after diluting the enzyme with ice-cold HBSS containing 5% FCS (FETAL CALF SERUM) and removal of the aggregates by settling for 2 min on ice. Aggregates were further digested with collagenase IV at 0.5 mg/ml for 10 min, and at 0.25 mg/ml for 6 min. Single cell suspensions were washed three times, and passed over a MASC Pre-separation filter (Miltenyi Biotec) to remove cell aggragetes and clumps. Single cell suspensions were incubated with anti-CD4 mAb-coated Microbeads (Miltenyi Biotec) and applied onto Mini-MACS columns (Miltenyi Biotec). This procedure yielded a positive fraction containing CD4⁺ cells. Immediately after purification cells were stimulated with PMA (PHORBOL MYRISTIC ACETATE) and ionomycin in complete medium, fixed, and stained the next day for intracellular production of cytokines.

### 5. Intracellular staining for cytokine production

Cells were stained for IFN-γ, IL-4, IL-10 and IL-2 as previously described (Trembleau, S., et al. Eur J Immunol. 1997, 27:2330-2339). Reagents for intracytoplasmic staining contained 1% FCS, 0.5% saponin (Sigma), and 0.1% sodium azide. All incubations were performed at room temperature. Cells were washed, pre-incubated for 10 min with PBS(phosphate buffered saline)/FCS/saponin, and then incubated with FITC(fluorescein isothiocyanate)-labeled rat anti-mouseIFN-γ (XMG1.2) and PE (phycoerythrin)-labeled rat anti-IL-2 (JE56-5H4), rat anti-mouse IL-4 (11B11), orPE-labeled rat anti-mouse IL-10 (JES5-16E3). Isotype controls were FITC- and PE-labeled rat IgG 1 (R3-34). After 30 min, cells were washed twice with PBS/FCS/saponin and then with PBS containing 5% of FCS without saponin to allow membrane closure. The cell surface was then stained with CyChrome-labeled anti-CD4 (L3T4) for 15 min at room temperature. Analysis was performed with a FACScan flow cytometer (Becton Dickinson, Mountain View, CA) equipped with CellQuest software, and 10,000 events were acquired.

### 6. Flow cytometric analysis

Single-cell suspensions of pancreas-infiltrating, spleen or lymph node cells were incubated with anti-CD4 mAb-coated Microbeads (Miltenyi Biotec) and applied onto Mini-MACS columns (Miltenyi Biotec). Stainings were performed in the presence of 100 µg/ml mouse IgG using the following mAbs, all from PharMingen: CyChrome-labeled Streptavidin, FITC-labeled anti-CD4 (L3T4), PE-labeled anti-CD45RB (16A), PE-labeled anti-CD62L (MEL-14), PE-labeled anti-CD38 (rat IgG2a), FITC-labeled anti-CD69 (H1.2F3), and biotinylated anti-CD25 (7D4). Cells were analyzed with a FACScan flow cytometer equipped with CellQuest software (Becton Dickinson, Mountain View, CA).

### 7. Cell cultures

Single cell suspensions from pancreatic lymph nodes or spleen were incubated with anti-CD4 mAb-coated Microbeads (Miltenyi Biotec) and applied onto Mini-MACS columns (Miltenyi Biotec). Purified CD4⁺ cells (6x 10⁵/well) were cultured for 48 h in roundbottom 96-well plates (Costar) pre-coated with 3 µg/ml purified anti-TCR mAb (ATCC HB 218; American Type Culture Collection, Manassas, VA) with or without 100 U/ml IL-2 and/or 10 µg/ml anti-CD28 mAb. Cultures were performed in synthetic HL-1 medium (Ventrex Laboratories, Portland, ME) supplemented with 2 mM L-glutamine and 50 µg/ml gentamicin (Sigma). To measure cell proliferation, cultures were pulsed 8 h before harvesting with 1 µCi [³H]TdR (40 Ci/nmol; Radiochemical Center, Amersham, U.K.). Incorporation of [³H]TdR was measured by liquid scintillation spectrometry. Alternatively, splenic or pancreatic lymph node CD4⁺CD25⁺ and CD4⁺CD25⁻ cells were sorted with MultiSort Kit (Miltenyi Biotec, Auburn, CA).

### 8. IL-12 and IFN-γ production in vivo

Lymphokine production was induced in 8 week-old female NOD mice by i.p. injection of 400 µg/mouse *Salmonella abortus equi* LPS (Sigma). Compound A was administered 72, 48, 24, and 1 h prior to LPS. Blood samples were collected 3 h post LPS injection and serum cytokines measured by ELISA.

### 9. Quantification of cytokines

Cytokines were quantified by two-sites ELISA. IFN-γ was determined using paired mAb from PharMingen (San Diego, CA). For capture, the mAb was AN-18.17.24. Samples were titrated and incubated overnight at 4°C. To detect bound IFN- y plates were then incubated with biotinylated XMG1.2 mAb. After washing, the bound biotinylated mAb was revealed by an additional 30-min incubation with alkaline phosphatase-conjugated streptavidin (Jackson ImmunoResearch Laboratories, Avondale, PA) diluted 1/5000. The plates were washed again and incubated with the developing substrate p-nitrophenylphosphate disodium (Sigma) in diethanolamine buffer (pH 9.6; 100 µl/well). The reaction was stopped by adding 50 µl/well NaOH 3N, and absorbance was read at 405 nm. ELISA for IL-12p75 was performed as described (Gately, M.K., et al., Current Protocols Immunol. 1995, 3, 15:6.16.11-16.16.15). Cytokines were quantified from two to three titration points using standard curves generated by purified recombinant mouse cytokines and results expressed as cytokine concentration in ng or pg/ml. Detection limits were 15 pg/ml for both cytokines.

### 10. Transfer experiment

Single cell suspensions were prepared from untreated 8 weeks-old NOD or Compound A treated NOD mice and adoptively transferred by intravenous injection into NOD-SCID mice (SCID means severe combined immunodeficiency). Alternatively, splenic or pancreatic lymph node CD4⁺CD25⁺CD38⁺ and CD4⁺CD25⁻CD38⁻ cells were sorted with MultiSort Kit (Miltenyi Biotec, Auburn, CA). Purified cells were mixed as indicated and adoptively transferred by intravenous injection into NOD-SCID mice. Glucose levels in the tail venous blood were quantified. A diagnosis of diabetes was after two sequential glucose measurements higher than 200 mg/dl.

### Example 1

### Inhibition of type 1 DM and insulitis development by Compound A administration

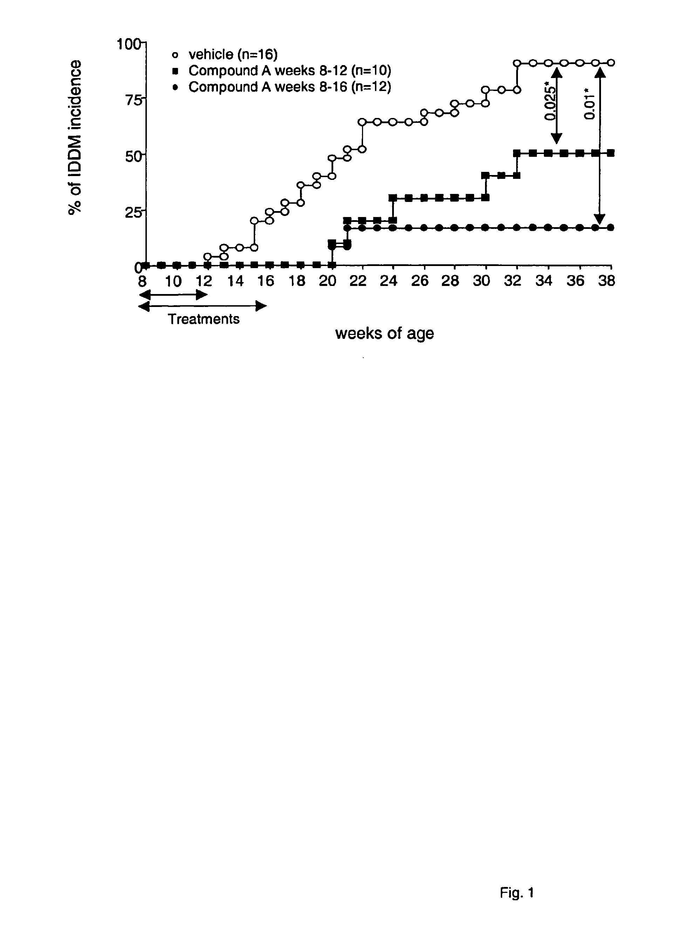

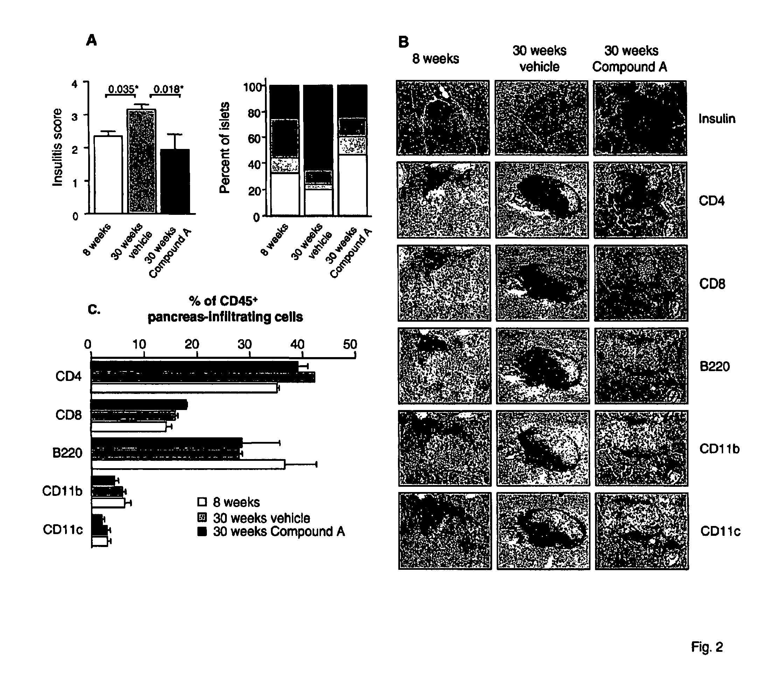

NOD female mice were treated daily with Compound A (0.03 µg/Kg body weight per os) or vehicle from 8 to 12 or 16 weeks of age and glycemia levels were monitored until 38 weeks of age. The incidence of disease was significantly lower in mice treated with Compound A compared to controls, and the longer treatment was most effective (Fig. 1). About 90% of vehicle-treated controls were diabetic by 38 weeks of age compared to only 50% of mice treated with Compound A from 8 to 12 weeks of age. A longer treatment, from 8 to 16 weeks of age, afforded higher protection, and only 16% of NOD mice developed type 1 DM. The score and severity of insulitis was similar between 8 weeks old and 1,25-dihydroxy-16,23Z-diene-26,27-hexafluoro-19-nor vitamin D₃-treated NOD mice at 30 weeks of age, whereas this had significantly progressed in vehicle-treated mice (Fig. 2A). These data indicate that Compound A treatment is able to halt the recruitment of infiltrating cells into the pancreatic islets. Functional islet β cells, demonstrated by the positive staining for insulin, were more frequent in both 8 week-old and 1,25-dihydroxy-16,23Z-diene-26,27-hexafluoro-19-nor vitamin D₃-treated mice at 30 weeks of age, compared to islets of vehicle-treated NOD mice at 30 weeks of age (Fig. 2B). Immuno-histochemical analysis showed a similar pattern of islet infiltration in 8 week-old and 1,25-dihydroxy-16,23Z-diene-26,27-hexafluoro-19-nor vitamin D₃-treated NOD mice at 30 weeks of age, comprising CD4⁺ and CD8⁺ T cells, B220⁺ B cells, CD11b⁺ macrophages, and CD11c⁺ dendritic cells. Conversely, a more florid infiltrate was observed in vehicle-treated 30 week-old NOD mice (Fig. 2B). The pancreas-infiltrating cells were analyzed by flow cytometry and no difference in the percentage of CD4⁺, CD8⁺, B220⁺ CD11b⁺, and CD11c⁺ cells was found among the three groups (Fig. 2C), indicating that Compound A treatment blocked the progression of insulitis without modifying the cellular composition of the infiltrate. In conclusion, NOD mice treated with Compound A from 8 to 16 weeks of age and analyzed at 30 weeks of age, when vehicle-treated mice presented over 60% of highly infiltrated islets, displayed a grade of insulitis similar to that found in untreated 8 week-old NOD mice, consistent with the reduction of type 1 DM development.

### Example 2

### Inhibition of LPS-induced IL-12 and IFN-γproduction by Compound A administration

The IFN-γ production induced by LPS administration is largely IL-12 dependent, and it has previously been shown that the LPS-induced serum levels of both cytokines can be inhibited by a short treatment with 1,25(OH)₂D₃ or its analogues (Mattner, F., et al. Eur J Immunol. 2000, 30:498-508). In this experiment the capacity of Compound A to inhibit LPS-induced IL-12 and IFN-γ production in vivo is tested. Mice were pre-treated with vehicle or with 0.03 µg/Kg Compound A administered per os daily for 4 days before LPS injection. Compound A did not induce hypercalcemia, as demonstrated by the serum calcium levels similar to controls, but inhibited significantly the LPS-induced IL-12p75 and IFN-γ production, with a potency about 100-fold higher than 1,25(OH)₂D₃ (Mattner, F., et al. Eur J Immunol. 2000, 30:498-508).

### Example 3

### Inhibition of IL-2 and IFN-γproduction in CD4⁺ pancreatic lymph node cells from Compound A treated NOD mice

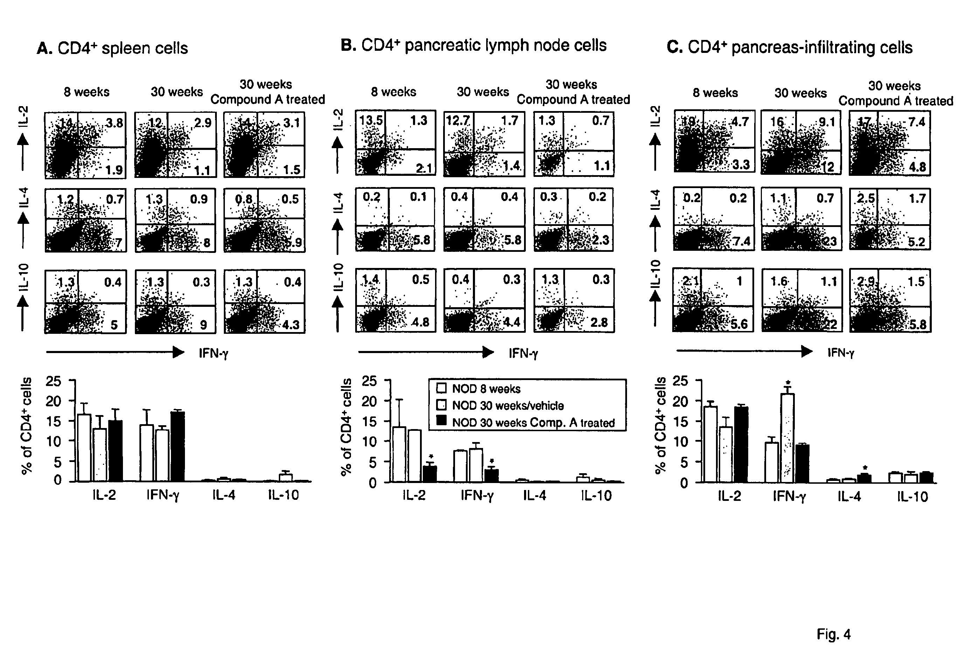

To identify mechanisms accounting for the reduced type 1 DM development in 1,25-dihydroxy-16,23Z-diene-26,27-hexafluoro-19-nor vitamin D₃-treated NOD mice, the cytokines produced by CD4⁺T cells in the spleen, pancreatic lymph node and pancreas-infiltrating cells of 8 and 30 week-old NOD mice, treated from 8 to 16 weeks of age with Compound A or vehicle were analyzed. Results in Fig. 4 demonstrate that splenic CD4⁺ T cells from 8 week-old and 30 week-old Compound A or vehicle-treated NOD mice displayed a similar cytokine production pattern. In contrast, analysis of CD4⁺ T cells from pancreatic lymph nodes revealed a 10-fold reduction of IL-2 producing cells and 3-fold reduction of IFN-γ-producing cells in 1,25-dihydroxy-16,23Z-diene-26,27-hexafluoro-19-nor vitamin D₃-treated compared to both 30 week-old vehicle-treated or 8 week-old NOD mice. CD4⁺ pancreas-infiltrating cells showed a similar percentage of IL-2-producing cells, but the IFN-γ-producing cells were significantly increased in vehicle-treated mice while they were similarly lower in 8 week-old and 1,25-dihydroxy-16,23Z-diene-26,27-hexafluoro-19-nor vitamin D₃-treated NOD mice.

These results indicate that Compound A was able to arrest the recruitment of IFN-γ-producing cells into the pancreas. No increase in IL-10-producing CD4⁺cells was observed and a modest, although significant, increase in IL-4-producing cells (means of three experiments 0.7 ± 0.2% in 8 weeks old and 0.9 ± 0.2% in vehicle-treated vs. 2.5 ± 0.3% in 1,25-dihydroxy-16,23Z-diene-26,27-hexafluoro-19-nor vitamin D₃-treated NOD mice P=0.02 by Mann-Whitney U-test) was detected.

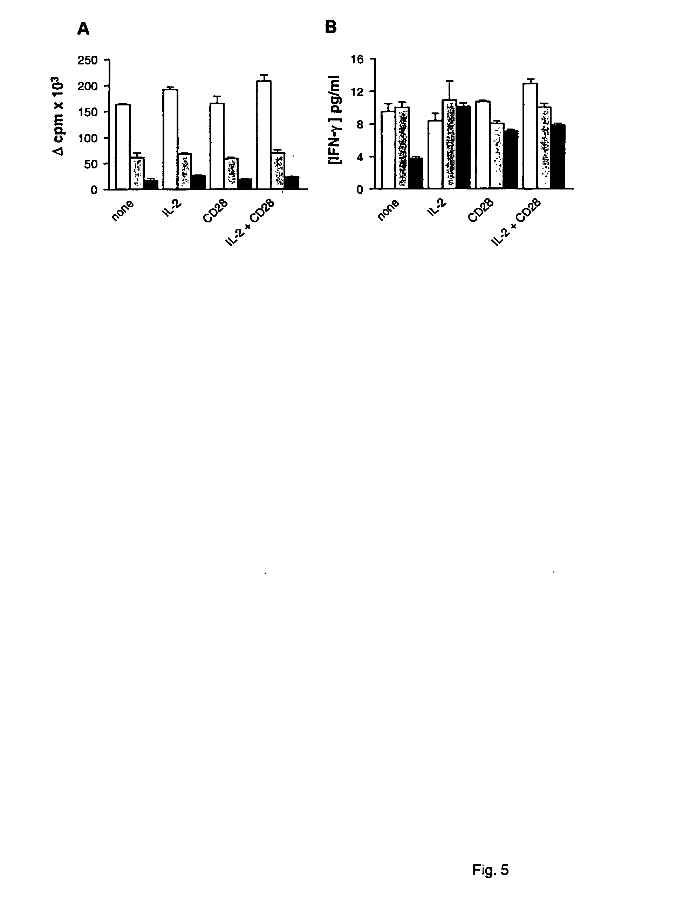

The profound reduction of IL-2-producing cells in 1,25-dihydroxy-16,23Z-diene-26,27-hexafluoro-19-nor vitamin D₃-treated NOD mice was paralleled by the strongly decreased proliferative response to insolubilized anti-TCR mAb. Pancreatic lymph node CD4⁺ cells from 20 week-old 1,25-dihydroxy-16,23Z-diene-26,27-hexafluoro-19-nor vitamin D₃-treated NOD mice proliferated four times less than age-matched controls and 10 times less compared to cells from 8 week-old mice (Fig. 5). The proliferation was not restored by the addition of IL-2, alone or together with anti-CD28 mAb, indicating a profoundly anergic state of these cells. However, addition of IL-2 and/or anti-CD28 mAb restored the lower IFN-γ secretion in cells from 1,25-dihydroxy-16,23Z-diene-26,27-hexafluoro-19-nor vitamin D₃-treated mice. In contrast to CD4⁺ cells from pancreatic lymph node, splenic CD4⁺ T cells isolated from 1,25-dihydroxy-16,23Z-diene-26,27-hexafluoro-19-nor vitamin D₃-treated mice or age-matched and 8 week-old controls, stimulated with insolubilized anti-TCR mAb, showed a similar proliferative response and IFN-γ production (data not shown). This indicates a selective effect of Compound A treatment on IL-2 and IFN-γ secretion by pancreatic lymph node CD4⁺ cells stimulated via TCR ligation, consistent with the results obtained by stimulation with PMA and ionomycin (Fig. 4).

### Example 4

### Compound A treatment enhances the frequency of CD4⁺CD25⁺ cells

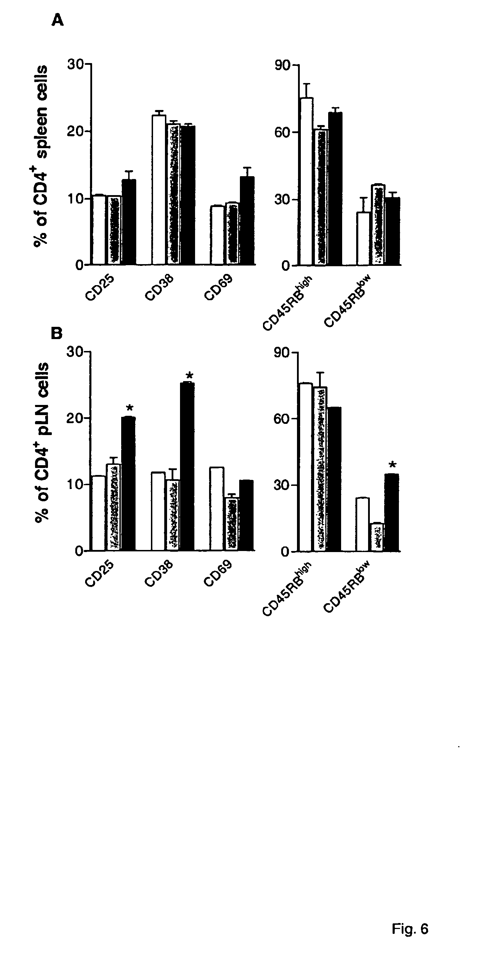

CD4⁺CD25⁺ T cells possess regulatory activity and have been implicated in the control of autoimmune responses in several models (Shevach, E.M.Annu Rev Immunol. 2000,18:423-449), including type 1 DM in NOD mice (Salomon, B., et al. Immunity. 2000, 12:431-440). Therefore, we analyzed the frequency of CD4⁺CD25⁺ cells in 1,25-dihydroxy-16,23Z-diene-26,27-hexafluoro-19-nor vitamin D₃-treated NOD mice. No difference was found in the percentage of splenic CD4⁺ cells expressing CD25, CD38, CD69, CD62L and CD45RB in 20 week-old Compound A or vehicle-treated and untreated 8 week-old NOD mice (Fig. 6A). In contrast, analysis of pancreatic lymph node cells revealed in 1,25-dihydroxy-16,23Z-diene-26,27-hexafluoro-19-nor vitamin D₃-treated compared to 8 week-old or vehicle-treated 20 week-old NOD mice, respectively, a significantly increased percentage of CD4⁺CD25⁺ (20.1 ± 0.1 % vs 11.6 ± 0.1 % or 13.0 ± 0.1, P <0.05 by Mann-Whitney U-test vs. 8 week-old mice) and CD4⁺CD38⁺ (25.2 ± 0.2 % vs. 11.8 ± 0.1 % or 10.6 ± 1.0, P <0.05 by Mann-Whitney U-test vs. 8 week-old mice). In addition, pancreatic lymph node cells from 1,25-dihydroxy-16,23Z-diene-26,27-hexafluoro-19-nor vitamin D₃-treated NOD mice contained a slightly lower percentage of CD45RB^{high} (65.1 ± 0.3 vs. 75.9 ± 0.5 % or 74.2 ± 1.6) and a significantly higher percentage of CD45RB^{low}CD4⁺ cells compared to 8 week-old and 20 week-old vehicle-treated NOD mice (24.1 ± 0.22 % vs. 12.65 ± 0.43 % or 34.8 ± 0.01, P <0.05 by Mann-Whitney U-test vs. 8 week-old mice) (Fig. 6B). Interestingly, double positive CD25⁺CD38⁺ CD4⁺ cells (17% vs. 8% or 7%) were selectively increased in the pancreatic lymph node of Compound A treated compared to 8 week-old and 20 week-old vehicle-treated NOD mice. These results indicate that Compound A administration leads to a selective increase of CD4⁺ cells with a regulatory phenotype in the pancreatic lymph node of NOD mice.

The results of the examples 1 to 4 demonstrate that administration of the 1,25(OH)₂D₃ analogue, 1,25-dihydroxy-16,23Z-diene-26,27-hexafluoro-19-nor vitamin D₃, to adult NOD mice blocks the progression of insulitis, and prevents type 1 DM development.

### Example 5

### Transplantation

Fully vascularized hearts were grafted into the abdomen of recipient mice as described (Corry, R. J., H. J. Winn, and P. S. Russell. Primarily vascularized allografts of hearts in mice. The role of H-2D, H-2K, and non-H-2 antigens in rejection. Transplantation. 1973, 16:343). Graft survival was monitored by palpation and rejection was confirmed by direct graft inspection. The results are compiled in Table A.

**Table A VASCULARIZED HEART C57BL/6 into BALB/c recipients**

| ***n*** | **Treatment** | **Dose** | **Graft survival (days)** | **Mean** | **SD** | **P value** |
|---|---|---|---|---|---|---|
| 6 | none | - | 8, 8, 9, 9, 10,10 | 9.00 | 0.9 | - |
| 6 | Reference | 5^{a} | 12, 12, 14, 15, 16, 17 | 14.33 | 2.1 | 0.0022 |
| 3 | Compound A | 0.01^{b}- 0.003^{c} | 12, 12, 12 | 12.00 | | ns |
| 4 | Compound A | 0.1^{b} - 0.03^{d} | 22, 27, 27, 77 | 38.20 | 25.9 | 0.0095 |

| | | | | | | |
|---|---|---|---|---|---|---|
| ^{a} Treatment per os (3x/w) every other day from d -1 until d +30 ^{b} µg/kg po (d-1,0,+1) ^{c} µg/kg po ( d +2, until d +30) ^{d} µg/kg (d+2 until d 15) | | | | | | |

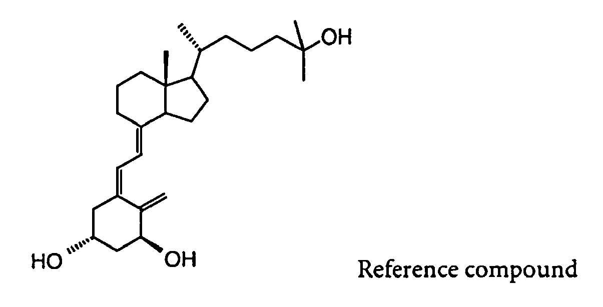

### Example 6

### Aortic Allograft

### Materials and Methods

Mice: BALB/c and C57BL/6 (B6) male mice, weighing 22-24 g, were purchased from Charles River Laboratories (Calco, Italy), and kept under specific pathogen-free conditions. B6 (H-2^{b}) mice were used as donors and BALB/c (H-2^{d}) as recipients of aortic allografts. Aorta transplantation: The entire procedure was performed according to Koulack et al. Development of a mouse aortic transplant model of chronic rejection Microsurgery 1995; 16: 110-3, with the modification described by Hong et al. Improved surgical technique for the establishment of a murine model of aortic transplantation. Microsurgery 1998; 18: 368-71. The donor thoracic aorta was harvested and divided into three segments of equivalent length, about 6 mm. The abdominal cavity was exposed by a transverse incision and the segment of donor thoracic aorta was anastomosed to the infra-renal portion, end-to-side, of the recipient abdominal aorta. All procedures were performed under isofluorane inhalation anesthesia using an stereomicroscope (M-3Z, Leica, Switzerland). Treatments: Compound A (1,25-dihydroxy-16,23Z-diene-26,27-hexafluoro-19-nor vitamin D₃) was dissolved in ethanol (1 mg/ml) and then diluted in miglyol. Mice were dosed orally with Compound A (0.03 µg/kg p.o. fives times per week), from day 0 to day 30 relative to the time of grafting. Control mice were dosed with vehicle only. Histology: Segments of aorta grafts were snap-frozen in Tissue Tek (Miles Laboratories, Elkhart, IN), and stored at -80°C. Frozen sections, 5 µm thick, were stained with hematoxylin and eosin, or with biotinylated mAbs against CD4, CD8, B220, CD1 1b or CD11c (all purchased from PharMingen, San Diego, CA), followed by streptavidin-peroxidase conjugate. 3-amino-9-ethylcarbazole (DAKO, Carpenteria, CA) was used as chromogen, and hematoxylin as a counterstain.

Determination of luminal reduction: Luminal reduction was assessed in 5 µm-thick frozen transverse aortic sections stained with hematoxylin and eosin obtained 60 days post transplantation. To quantify morphological changes in the graft, we used a Leica DC camera (Leica IM 1000 image manager) to acquire the image, and the Leica Qwin software to calculate the luminal reduction percentage. This was defined as (area bounded by the internal elastic lamina minus area of the lumen) x100 divided by the area bounded by the internal elastic lamina.

The average values for each analysis were obtained by averaging the results obtained from 3 sections for each allograft.

Statistical analysis: Numerical data are expressed as mean ± SD. Data on percent luminal reduction were analysed by a non parametric Students's t-test.

### Results

The effect of Compound A in preventing chronic rejection was evaluated using the aortic graft model in the mouse. Graft combinations were syngeneic or allogeneic. In allogeneic combinations, recipient mice were treated orally with vehicle alone or containing Compound A (0.03 µg/kg) fives times per week, from day 0 to day 30 relative to the time of grafting. Aortic grafts were analyzed 60 days after transplantation.

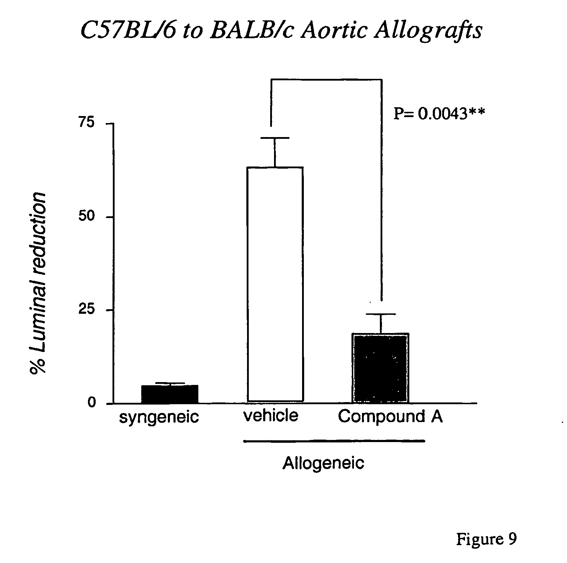

Syngeneic BALB/c to BALB/c aortic grafts developed negligible intimal thickening (< 5%), indicating lack of chronic rejection (Fig. 9). BALB/c aortic grafts transplanted into B6 recipients treated with vehicle only showed a well-established intimal thickening (> 60%). This was significantly decreased (<20%) in Compound A treated mice (Fig. 9).

The allogeneic aortic segments from vehicle-treated recipients showed an important inflammatory cell response, with a relatively high number of macrophages and dendritic cells, but fewer CD4, CD8 and B cells, infiltrating mainly the adventitia layer. In marked contrast, the grafts from mice treated with Compound A showed a profoundly reduced number of macrophages and dendritic cells, and complete absence of CD4, CD8 and B cells.

In general, the Compound A may be administered in amounts between about 0.001 µg/Kg body weight to about 0.5 µg/Kg body weight per day, preferably from about 0.005 to about 0.1 µg/Kg body weight per day, most preferably from about 0.01 to about 0.05 µg/Kg body weight per day. This dosage may be delivered in a conventional pharmaceutical composition by a single administration, by multiple applications, or via controlled release, as needed to achieve the most effective results, preferably once or twice daily by mouth. In certain situations, alternate day dosing may prove adequate to achieve the desired therapeutic response.

The selection of the exact dose and composition and the most appropriate delivery regimen will be influenced by, inter alia, the pharmacological properties of the formulation, the nature and severity of the condition being treated, and the physical condition and mental acuity of the recipient.

Representative delivery regimens include oral, parenteral (including subcutaneous, intramuscular and intravenous), rectal, buccal (including sublingual), pulmonary, transdermal, and intranasal, most preferably oral. Administration may be continuous or intermittent (e.g., by bolus injection).

A further aspect of the present invention relates to pharmaceutical compositions comprising as an active ingredient a compound of the present invention, in admixture with a pharmaceutically acceptable, non-toxic carrier. As mentioned above, such compositions may be prepared for parenteral (subcutaneous, intramuscular or intravenous) administration, particularly in the form of liquid solutions or suspensions; for oral or buccal administration, particularly in the form of tablets or capsules; for pulmonary or intranasal administration, particularly in the form of powders, nasal drops or aerosols; and for rectal or transdermal administration.

The compositions may conveniently be administered in unit dosage form and may be prepared by any of the methods well-known in the pharmaceutical art, for example as described in Remington's Pharmaceutical Sciences, 17th ed., Mack Publishing Company, Easton, PA., (1985). Formulations for parenteral administration may contain as excipients sterile water or saline, alkylene glycols such as propylene glycol, polyalkylene glycols such as polyethylene glycol, oils of vegetable origin, hydrogenated naphthalenes and the like. Formulations for nasal administration may be solid and may contain excipients, for example, lactose or dextran, or may be aqueous or oily solutions for use in the form of nasal drops or metered spray. For buccal administration typical excipients include sugars, calcium stearate, magnesium stearate, pregelatinated starch, and the like.

Orally administrable compositions may comprise one or more physiologically compatible carriers and/or excipients and may be in solid or liquid form. Tablets and capsules may be prepared with binding agents, for example, syrup, acacia, gelatin, sorbitol, tragacanth, or poly-vinylpyrollidone; fillers, such as lactose, sucrose, corn starch, calcium phosphate, sorbitol, or glycine; lubricants, such as magnesium stearate, talc, polyethylene glycol, or silica; and surfactants, such as sodium lauryl sulfate. Liquid compositions may contain conventional additives such as suspending agents, for example sorbitol syrup, methyl cellulose, sugar syrup, gelatin, carboxymethylcellulose, or edible fats; emulsifying agents such as lecithin, or acacia; vegetable oils such as almond oil, coconut oil, cod liver oil, or peanut oil; preservatives such as butylated hydroxyanisole (BHA) and butylated hydroxytoluene (BHT). Liquid compositions may be encapsulated in, for example, gelatin to provide a unit dosage form.

Preferred solid oral dosage forms include tablets, two-piece hard shell capsules and soft elastic gelatin (SEG) capsules. SEG capsules are of particular interest because they provide distinct advantages over the other two forms (see Seager, H., "Soft gelatin capsules: a solution to many tableting problems"; Pharmaceutical Technology, 9, (1985). Some of the advantages of using SEG capsules are: a) dose-content uniformity is optimized in SEG capsules because the drug is dissolved or dispersed in a liquid that can be dosed into the capsules accurately b) drugs formulated as SEG capsules show good bioavailability because the drug is dissolved, solubilized or dispersed in an aqueous-miscible or oily liquid and therefore when released in the body the solutions dissolve or are emulsified to produce drug dispersions of high surface area and c) degradation of drugs that are sensitive to oxidation during long-term storage is prevented because the dry shell of soft gelatin provides a barrier against the diffusion of oxygen.

The dry shell formulation typically comprises of about 40% to 60% concentration of gelatin, about a 20% to 30% concentration of plasticizer (such as glycerin, sorbitol or propylene glycol) and about a 30 to 40% concentration of water. Other materials such as preservatives, dyes, opacifiers and flavours also may be present. The liquid fill material comprises a solid drug that has been dissolved, solubilized or dispersed (with suspending agents such as beeswax, hydrogenated castor oil or polyethylene glycol 4000) or a liquid drug in vehicles or combinations of vehicles such as mineral oil, vegetable oils, triglycerides, glycols, polyols and surface-active agents.

### Example: Oral Dosage Form Soft Gelatin Capsule

A capsule for oral administration is formulated under nitrogen in amber light from 0.01 to 25.0 mg of one of the compounds of the present invention in 150 mg of fractionated coconut oil, with 0.015 mg butylated hydroxytoluene (BHT) and 0.015 mg butylated hydroxyanisole (BHA), filled in a soft gelatin capsule.

### Figures

Figure 1. (page 1/9) Compound A administration to 8-week-old NOD mice inhibits type 1 DM development. NOD mice were treated 5x/week with vehicle (open circles) or with 0.03 µg/Kg Compound A p.o. (filled circles) from 8 to 16 weeks of age, or from 8 to 12 weeks of age (filled squares). Type 1 DM development was monitored twice weekly by measurement of blood glucose levels. The P values were calculated by Mann-Whitney U test.

Figure 2. (page 2/9) Decreased insulitis in Compound A -treated NOD mice. A. Histological scoring of insulitis was performed on pancreas sections stained with hematoxylin/eosin from untreated 8 week-old, and NOD mice at 30 weeks of age treated with Compound A or vehicle from 8 to 16 weeks of age. Each bar represents the mean score of about 40-50 islets/mouse (*left panel*). The results refer to the mean values ± SE of 8 mice/group, except for 1,25-dihydroxy-16,23Z-diene-26,27-hexafluoro-19-nor vitamin D₃-treated mice where three mice were scored. The P values were calculated by Mann-Whitney U test. The severity of the infiltrate is shown in the *right panel.* Islets from the same mice were scored for absence of insulitis (□), peri-insulitis (□ ), moderate insulitis with <50% infiltration of the islets ( 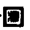 ), and severe insulitis, with >50% of infiltration of the islets (■).

B. Consecutive sections of the same islets were stained with anti-CD4, anti-CD8, anti-B220, anti-CD11b (Mac-1), and anti-CD11c (N418) mAbs. In addition, islets were stained for insulin. C. Cytofluorimetric analysis of pancreas-infiltrating cells. Pancreas-infiltrating cells isolated from untreated 8 week-old NOD mice, vehicle-treated and Compound A treated NOD mice at 30 weeks of age were stained with mAbs specific for the indicated surface molecules and analyzed by flow cytometry. Acquisition was performed on CD45⁺ cells. Bars represent the mean ± SE from three separate experiments.

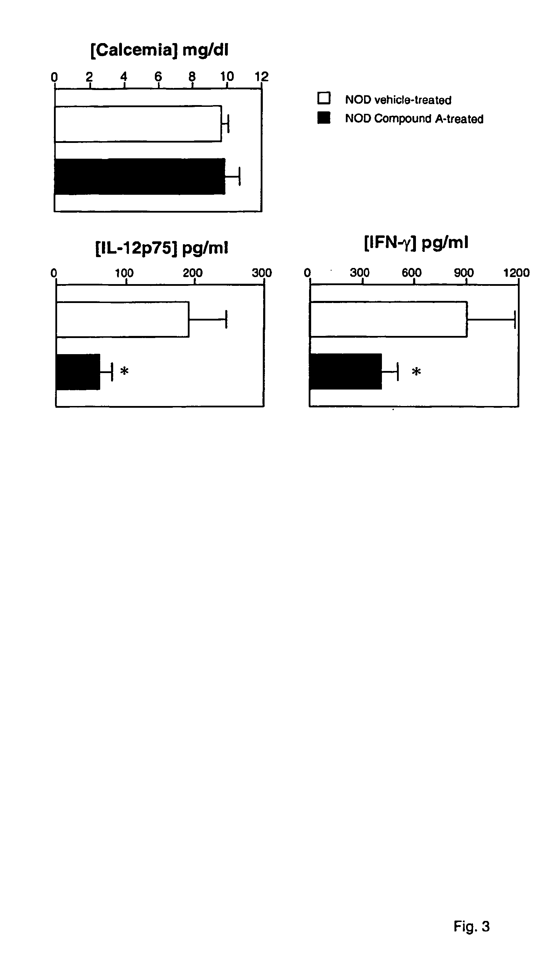

Figure 3. (page 3/9) Compound A treatment inhibits IL-12-dependent IFN-γ production *in vivo* without inducing hypercalcemia. Compound A (0.03 µg/Kg) was administered orally in miglyol to NOD mice 72, 48, 24 and 1 h before LPS injection. IFN-γ, IL-12p75 and serum calcium levels were quantified 3 h post LPS injection. Bars represent the mean ± SE of 10 mice/group. The P values were calculated by Mann-Whitney U test (* indicates P<0.05 vs. vehicle-treated NOD mice).

Figure 4. (page 4/9) Cytokine production by CD4⁺ cells from spleen, pancreatic lymph node and pancreas-infiltrating cells. Spleen (A), pancreatic lymph node (B) and pancreas-infiltrating (C) CD4⁺ cells were obtained from untreated 8 week-old or 20 week-old NOD mice treated 5x/week p.o. from 8 to 16 weeks of age with vehicle or with 0.03 µg/Kg Compound A. Positively selected CD4⁺ cells (2 x 10⁵ cells/well) were stimulated with PMA and ionomycin and analyzed by flow cytometry for IFN-γ (abscissa) and IL-2, IL-4 or IL-10 (ordinate) production. Acquisition was performed on CD4⁺ cells. Percentage of positive cells, set according to the isotype-matched controls (not shown), are shown in the top corner of each quadrant. Dot plots are from a representative experiment out of four performed. Bar graphs represent the mean ± SE of four independent experiments using pooled cells from 3 to 4 mice. The P values were calculated by Mann-Whitney U test (* indicates P<0.05 vs. 8 week-old NOD mice).

Figure 5. (page 5/9) Proliferation and IFN-γ secretion following TCR ligation. Splenic and pancreatic lymph node CD4⁺ cells from untreated 8 week-old (□) or 20 week-old NOD mice treated 5x/week from 8 to 16 weeks of age with vehicle (□) or with 0.03 µg/KgCompound A (■) were positively selected and stimulated (6 x 10⁵ cells/well) with plate-bound anti-TCR mAb (10 µg/ml) in the presence of 100 U/ml IL-2 and/or 5 µg/ml anti-CD28 mAb. A. Cells were pulsed with [³H]-thymidine during the last 8 hrs of a 48 h culture. Data are presented as mean ± SE of thymidine incorporation (Δ cpm) from triplicate cultures, background values were 790 ±100 cpm. B. After 48 h of culture, IFN-γ secretion was quantified by ELISA. Results, presented as mean ± SE of triplicate cultures, are from one representative experiment out of four performed.

Figure 6. (page 6/9) Compound A treatment enhances the frequency of CD4⁺CD25⁺ cells. Positively selected CD4⁺ T cells were stained with mAbs specific for the indicated surface molecules and analyzed by flow cytometry. Acquisition was performed on CD4⁺ cells. Bars represent the percent of spleen and lymph node CD4⁺T cells expressing the indicated surface molecules from untreated 8 week-old (□) or 20 week-old NOD mice treated 5x/week from 8 to 16 weeks of age with vehicle (□) or with 0.03 µg/Kg Compound A (■). Data are presented as mean ± SE of three separate experiments. The P values were calculated by Mann-Whitney U test (* indicates P<0.05 vs. 8 week-old NOD mice).

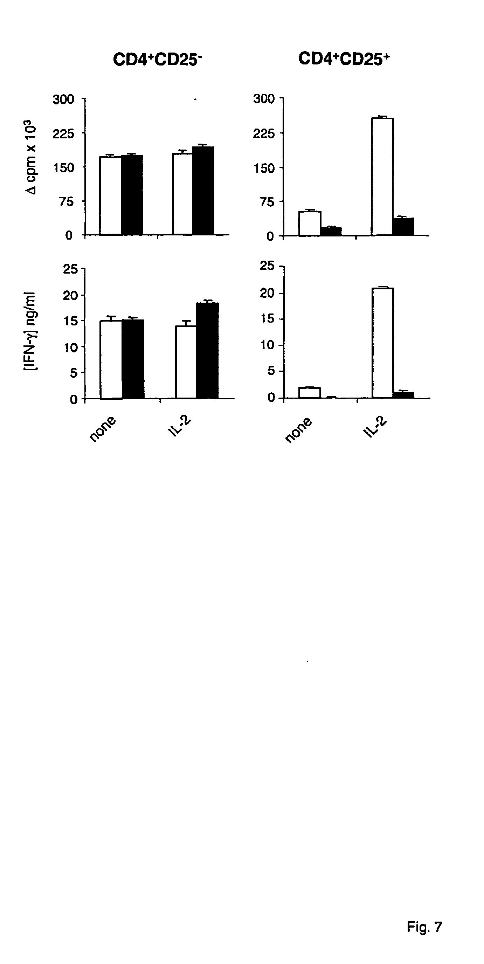

Figure 7. (page 7/9) Anergy of CD4⁺CD25⁺ T cells from 1,25-dihydroxy-16,23Z-diene-26,27-hexafluoro-19-nor vitamin D₃-treated NOD mice. Positively selected CD4⁺CD25⁺ and CD4⁺CD25⁻ cells (6 x 10⁵ cells/well) from untreated 8 week-old (□) or 20 week-old NOD mice treated 5x/week from 8 to 16 weeks of age with 0.03 µg/Kg Compound A (■) were stimulated with insolubilized anti-TCR mAb (10 µg/ml) with or without 100 U/ml IL-2. Cells were pulsed with [³H]-thymidine during the last 8 hrs of a 48 h culture. Data are presented as mean ± SE of thymidine incorporation (Δ cpm) from triplicate cultures, background values were 643 ± 65 cpm. B. After 48 h of culture, IFN-γ secretion was quantified by ELISA and expressed as mean ± SE from triplicate cultures. Results are from one representative experiment out of three performed.

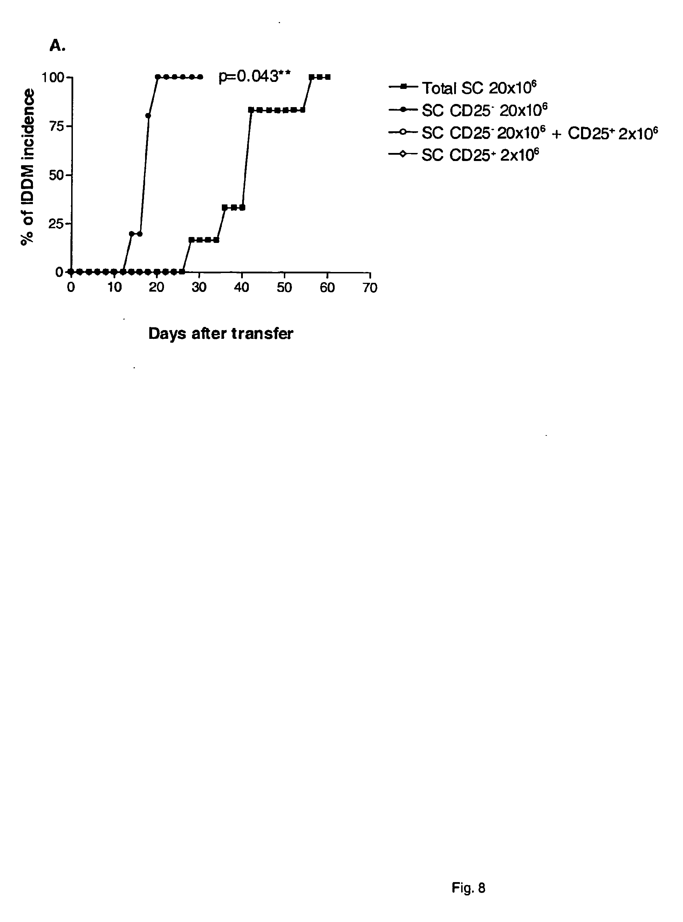

Figure 8. (page 8/9) CD4⁺CD25⁺ regulatory T cells control diabetes. A. Eight- to ten-week-old NOD-scid mice were injected with 2x10⁷ splenocytes from pre-diabetic NOD mice (closed square, n=10 ), 2x10⁷ splenocytes CD25⁺-depleted (closed circle, n=5 ), and coinjected with 2x10⁷ splenocytes CD25⁺-depleted and 2x10⁶ CD4⁺CD25⁺ splenocytes (open circle, n=5). Blood glucose levels were checked twice weekly.

Figure 9. (page 9/9) C57BL/6 to BALB/c Aortic Allografts. Syngeneic BALB/c to BALB/c aortic grafts developed negligible intimal thickening (< 5%), indicating lack of chronic rejection. BALB/c aortic grafts transplanted into B6 recipients treated with vehicle only showed a well-established intimal thickening (> 60%). This was significantly decreased (<20%) in Compound A treated mice.

## Claims

1. The use of 1,25-dihydroxy-16,23Z-diene-26,27-hexafluoro-19-nor vitamin D₃ for the manufacture of a medicament for preventing the progression of autoimmune diabetes.

2. The use according to claim 1, wherein the autoimmune diabetes is type 1 diabetes.

3. The use according to either claim 1 or claim 2, wherein 1,25-dihydroxy-16,23Z-diene-26,27-hexafluoro-19-nor vitamin D₃ is used in combination with an additional immunosuppressive agent.

4. The use of 1,25-dihydroxy-16,23Z-diene-26,27-hexafluoro-19-nor vitamin D₃ for the manufacture of a medicament for the prevention and/or treatment of acute and chronic allograft rejection.

5. The use according to claim 4, wherein 1,25-dihydroxy-16,23Z-diene-26,27-hexafluoro-19-nor vitamin D₃ is used in combination with an additional immunosuppressive agent.

## Patentansprüche

1. Verwendung von 1 ,25-Dihydroxy-16,23Z-dien-26,27-hexafluor- 19-nor-Vitamin D₃ zur Herstellung eines Medikaments zur Verhinderung des Fortschreitens von Autoimmundiabetes.

2. Verwendung nach Anspruch 1, wobei der Autoimmundiabetes Diabetes Typ 1 ist.

3. Verwendung nach entweder Anspruch 1 oder Anspruch 2, wobei 1,25-Dihydroxy-16.23Z-dien-26,27-hexafluor-19-nor-Vitamin D₃ in Kombination mit einem weiteren Immunosuppressivum verwendet wird.

4. Verwendung von 1,25-Dihydroxy-16,23Z-dien-26,27-hexafluor-19-nor-Vitamin D₃ zur Herstellung eines Medikaments zur Verhinderung und/oder Behandlung akuter und chronischer Transplantatabstoßung.

5. Verwendung nach Anspruch 4, wobei 1,25-Dihydroxy-16,23Z-dien-26,27-hexafluor-19-nor-Vitamin D₃ in Kombination mit einem weiteren Immunosuppressivum verwendet wird.

## Revendications

1. Utilisation de vitamine D₃ de 1,25-dihydroxy-16 ,23Z-diène-26,27-hexafluoro-19-nor pour la fabrication d'un médicament destiné à empêcher l'évolution du diabète chronique actif.

2. Utilisation selon la revendication 1, dans laquelle le diabète chronique actif est un diabète de type 1.

3. Utilisation selon la revendication 1 ou la revendication 2, dans laquelle la vitamine D₃ de 1,25-dihydroxy-16,23Z-diène-26,27-hexafluoro-19-nor est utilisée en combinaison avec un immunodépresseur supplémentaire.

4. Utilisation de vitamine D₃ de 1,25-dihydroxy-16,23Z-diène-26,27-hexafluoro-19-nor pour la fabrication d'un médicament destiné à la prévention/au traitement du rejet d'allogreffe chronique et de crise.

5. Utilisation selon la revendication 4, dans laquelle la vitamine D₃ de 1,25-dihydroxy-16,23Z-diène-26,27-hexafluoro-19-nor est utilisée en combinaison avec un immunodépresseur supplémentaire.
